# EUROPÄISCHE PATENTSCHRIFT

(11) **EP 0 369 397 B1**
(45) Veröffentlichungstag und Bekanntmachung des Hinweises auf die Patenterteilung: **22.09.1993**
(21) Anmeldenummer: 89121061.9
(22) Anmeldetag: 14.11.1989
(51) Int. Cl.: A61F 13/10

(54) **Elastische Handgelenkbandage**
Elastic wrist bandage
Bandage élastique pour poignet

(30) Priorität: 14.11.1988 DE 3838564
(43) Veröffentlichungstag der Anmeldung: 23.05.1990
(73) Patentinhaber: Bauerfeind GmbH & Co., D-47880 Kempen (DE)
(72) Erfinder: Hess, Heinrich, Prof. Dr. med., D-6630 Saarlouis (DE); Krause, Wolfgang, Prof. Dr. med., D-3500 Kassel (DE); Bauerfeind, Hans B., D-4152 Kempen 1 (DE)
(74) Vertreter: Bardehle, Heinz, Dipl.-Ing.

(56) Entgegenhaltungen:
- DE-A- 3 631 253
- US-A- 2 388 330
- US-A- 4 584 993

## Beschreibung

Die Erfindung bezieht sich auf eine elastische Handgelenkbandage in Schlauchform mit einer Daumenöffnung, mit eingearbeitetem Stützelement.

Eine derartige Handgelenkbandage ist beispielsweise aus dem DE-A-3 631 253 bekannt. Derartige Handgelenkbandagen werden zur Stützung des Handgelenks in großem Umfang verwendet.

Der Erfindung liegt die Aufgabe zugrunde, eine derartige Handgelenkbandage dahingehend zu verbessern, daß sie im wesentlichen ausschließlich auf ihre Stützfunktion konzentriert wird. Erfindungsgemäß geschieht dies dadurch, daß als Stützelement eine profilierte Polsterung vorgesehen ist, die den mittleren Bereich von Handrücken und Handfläche jeweils mit Übergang zum Unterarm ausspart, wobei das Profil der Polsterung so in den Randbereich zu den Aussparungen übergeht, daß das Bandagenmaterial sich im Bereich der Aussparungen unter nur leichtem Druck an die Weichteile anlegt.

Eine von der Bandage auf das Handgelenk ausgehende Druckwirkung wird auf diese Weise auf die gegenüber Druck weitgehend unempfindlichen Bereiche neben dem Handrücken und neben der Handfläche konzentriert. Es handelt sich dabei im wesentlichen um die Schmalseiten der Hand, des Handgelenks und des anschließenden Unterarmbereichs, die aufgrund ihrer anatomischen Gestaltung wesentlich weniger druckempfindlich sind als die Bereiche von Handrücken und Handfläche. Außerdem wird durch die Polsterung der Druck auf diese Schmalseiten weitgehend vergleichmäßigt, so daß er nicht unangenehm empfunden wird.

Durch die Aussparung des mittleren Bereichs von Handrücken und Handfläche von der Wirkung der Polsterung zur Erzeugung eines nur leichten Druckes wird unter Aufrechterhaltung der Stützfunktion der Bandage aufgrund der profilierten Polsterung erreicht, daß einerseits Blutgefäße und andererseits Nervenstränge praktisch druckfrei gehalten werden, so daß das Handgelenk hinsichtlich seiner Blut- und Nervenversorgung nicht nachteilig beeinflußt wird. Es liegen nämlich die durch Druck beeinflußbaren Blutgefäße weitgehend im mittleren Bereich des Handrückens und die Nervenstränge im mittleren Bereich der Handfläche. Dies ist bei den bisher bekannten Handgelenkbandagen nicht berücksichtigt worden, so daß bei ihrer Benutzung bei erzielter Stützfunktion als unerwünschte Nebenfunktion eine Blutabsperrung und eine Beeinträchtigung der Nervenfunktion entstand.

Zweckmäßig bildet man die Polsterung aus je einem entlang jeder Gelenkschmalseite in die Bandage eingearbeiteten Streifen aus elastischem Material aus, der als Pfeiler das brückenartig den Handrücken und die Handfläche überspannende Bandagenmaterial stützt. Derartige Streifen lassen sich in günstiger Weise mit dem Bandagenmaterial verarbeiten, beispielsweise durch Aufkleben oder Aufschweißen. Die Streifen aus elastischem Material, z. B. Silikonkautschuk, bewirken aufgrund ihrer Materialeigenschaften einen Ausgleich des auf die Gelenkschmalseiten ausgeübten Druckes, so daß hierdurch als unangenehm empfundene Druckstellen weitgehend vermieden werden. Darüber hinaus kann dieser Effekt noch dadurch intensiviert werden, daß die Polsterung an ihrer der Körperfläche zugewandten Seite Knochenvorsprünge aussparende Vertiefungen aufweist. Diese Vertiefungen werden vorzugsweise im Bereich der Griffelfortsätze von Elle und Speiche vorgesehen.

In den Figuren ist ein Ausführungsbeispiel der Erfindung dargestellt. Es zeigen:
- Figur 1: die auf eine Hand aufgezogene Handgelenkbandage, auf die flache Seite der Hand gesehen,
- Figur 2: einen Schnitt längs der Linie von II-II aus Figur 1.

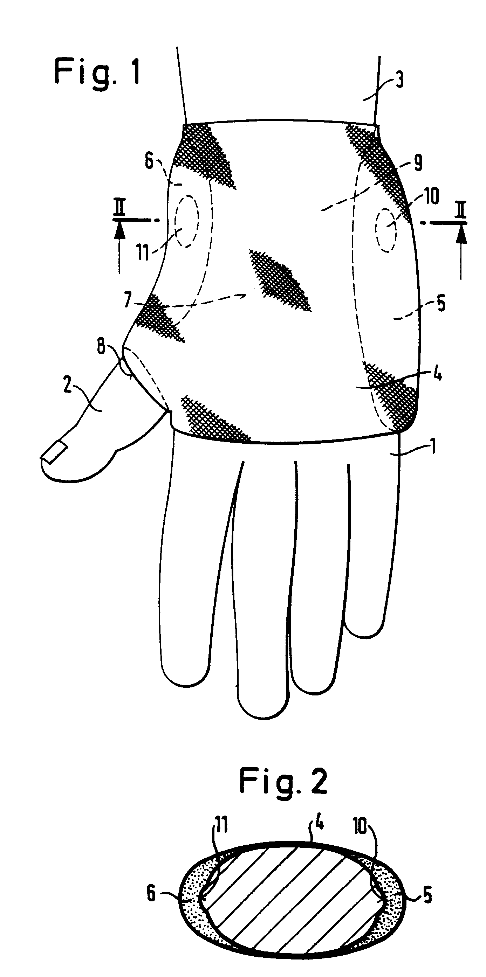

In Figur 1 ist eine menschliche Hand 1 mit Daumen 2 und Unterarm 3 dargestellt, wobei über das Handgelenk 9 eine elastische Handgelenkbandage 4 gezogen ist, die sich über das eigentliche Handgelenk 9 bis in die Mittelhand und den an das Handgelenk anschließenden Teil des Unterarms 3 erstreckt. In der Figur 1 wird zwischen Handrücken und Handfläche nicht unterschieden, da es sich bei der dargestellten Hand sowohl um eine rechte als auch eine linke handeln kann. Die Handgelenkbandage 4 ist für den Durchtritt des Daumens 2 mit einer Daumenöffnung 8 versehen. Die Handgelenkbandage 4 besteht aus einem bekannten elastischem Textilmaterial. An den Gelenkschmalseiten ist je eine Polsterung als Streifen 5 und 6 eingearbeitet, der sich im wesentlichen längs der gesamten Handgelenkbandage erstreckt, jedoch die mittleren Bereiche von Handfläche bzw. Handrücken 7 ausspart.

Die Form der als profilierte Polsterung dienenden Streifen 5 und 6 geht aus der Figur 2 hervor. Diese zeigt einen Schnitt längs der Linie II-II aus Figur 1. Demgemäß umschließen die Streifen 5 und 6 die jeweilige Gelenkschmalseite im Querschnitt sichelförmig derart, daß die die auslaufenden Enden des sichelförmigen Querschnitts brückenartig verbindenden Bereiche der Bandage 4 unter nur leichtem Druck an die Weichteile des Handgelenks 9 und der anschließenden Körperteile anliegen.

Aufgrund dieser Gestaltung und Profilierung der als Polsterung dienenden Streifen 5 und 6 ergibt sich ein seitlicher Druck auf die beiden Gelenkschmalseiten, ohne daß ein nennenswerter Druck auf die mittleren Bereiche von Handrücken bzw. Handfläche 7 ausgeübt wird. Infolgedessen bleiben beim Tragen dieser Handgelenkbandage 6 die Blutzirkulation und die Nervenfunktion weitgehend unbeeinträchtigt.

Aufgrund der Gestaltung der Streifen 5 und 6 aus elastischem Material verteilen diese gleichmäßig den Druck auf die Gelenkschmalseiten, was einerseits der Stützfunktion der Handgelenkbandage 6 zugute kommt, andererseits das Entstehen von unangenehm empfundenen Druckstellen vermeidet. Darüber hinaus sind bei dem Ausführungsbeispiel gemäß Figur 1 und 2 in die Streifen 5 und 6 noch die Vertiefungen 10 und 11 vorgesehen, die Knochenvorsprünge an Elle und Speiche aufnehmen.

## Patentansprüche

1. Elastische Handgelenkbandage (4) in Schlauchform mit einer Daumenöffnung (8), mit eingearbeitetem Stützelement,
**gekennzeichnet dadurch**, daß als Stützelement eine profilierte Polsterung (5,6) vorgesehen ist, die den mittleren Bereich von Handrücken und Handfläche (7) einschließlich den anschließenden Unterarmteilen ausspart, wobei das Profil der Polsterung (5,6) so in den Randbereich zu den Aussparungen übergeht, daß das Bandagenmaterial sich im Bereich der Aussparungen unter nur leichtem Druck an die Weichteile anlegt.

2. Handgelenkbandage nach Anspruch 1, dadurch gekennzeichnet, daß die Polsterung aus je einem entlang jeder Gelenkschmalseite in die Bandage eingearbeiteten Streifen (5,6) aus elastischem Material besteht, der als Pfeiler das brückenartig den Handrücken und die Handfläche (7) überspannende Bandagenmaterial stützt.

3. Handgelenkbandage nach Anspruch 1 oder 2, dadurch gekennzeichnet, daß die Polsterung (5,6) an ihrer Innenseite Knochenvorsprünge aussparende Vertiefungen (10,11) aufweist.

## Claims

1. Elastic wrist bandage (4) in tubular form with a thumb opening (8) and an incorporated supporting element, characterised in that profiled padding (5, 6) which does not touch the central region of the back of the hand and the palm (7), including the adjoining parts of the forearm, is provided as a supporting element, the profile of the padding (5, 6) merging into the unpadded portions in the edge region in such a manner that the bandage material rests with only light pressure against the soft parts in the region of the unpadded portions.

2. Wrist bandage according to claim 1, characterised in that the padding consists of strips (5, 6) of elastic material incorporated into the bandage along each narrow side of the joint, these acting as uprights to support the bandage material spanning the back of the hand and the palm (7) like a bridge.

3. Wrist bandage according to claim 1 or claim 2, characterised in that the padding (5, 6) is provided on its inner face with recesses (10, 11) leaving room for bony prominences.

## Revendications

1. Bandage élastique (4) pour poignet, de forme tubulaire, avec une ouverture (8) pour le pouce et avec un élément de soutien incorporé, **caractérisé** en ce qu'il est prévu comme élément de soutien un rembourrage profilé (5, 6), qui épargne la région centrale du dos de la main et de la paume (7), y compris la partie limitrophe de l'avantbras, le profil du rembourrage (5, 6) se raccordant aux parties épargnées, dans la région marginale, de telle sorte que le matériau du bandage ne s'applique contre les parties molles, dans les régions épargnées par le rembourrage, qu'avec une légère pression.

2. Bandage élastique pour poignet selon la revendication 1, **caractérisé** en ce que le rembourrage est constitué de bandes de matériau élastique (5, 6), qui sont respectivement incorporées dans le bandage le long de chaque tranche du poignet et soutiennent, à la manière de piliers, le matériau du bandage recouvrant à la manière d'un pont le dos de la main et la paume (7).

3. Bandage élastique pour poignet selon la revendication 1 ou 2, **caractérisé** en ce que le rembourrage (5, 6) présente, sur son côté intérieur, des renfoncements (10, 11) épargnant les saillies osseuses.
